(19)

Europäisches
Patentamt
European
Patent Office
Office européen
des brevets

(11) **EP 4 151 217 A1**

(12) **EUROPEAN PATENT APPLICATION**

(43) Date of publication:
**22.03.2023 Bulletin 2023/12**

(21) Application number: **21196947.2**

(22) Date of filing: **15.09.2021**

(51) International Patent Classification (IPC):
*A61K 31/505* (2006.01)   *A61K 35/741* (2015.01)
*A61K 35/747* (2015.01)   *A61P 17/00* (2006.01)
*A61P 17/04* (2006.01)   *A61P 17/06* (2006.01)
*A61P 17/08* (2006.01)   *A61P 17/10* (2006.01)
*A61K 8/36* (2006.01)   *A61K 8/49* (2006.01)

(52) Cooperative Patent Classification (CPC):
(C-Sets available)
**A61Q 19/00; A23L 33/135; A61K 8/4953;
A61K 8/99; A61K 31/505; A61K 35/741;
A61K 35/747; A61P 17/00; A61P 17/04;
A61P 17/06; A61P 17/08; A61P 17/10;**
A23V 2002/00; A61K 2800/884; A61K 2800/92

(Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Alexander Werz S.R.L.
50019 Sesto Fiorentino (FI) (IT)**

(72) Inventors:
• **MALANCHIN, Rosella
21040 ORIGGIO (VA) (IT)**
• **CARLOMAGNO, Federica
21040 ORIGGIO (VA) (IT)**

(74) Representative: **Bianchetti & Minoja SRL
Via Plinio, 63
20129 Milano (IT)**

(54) **COMBINED TOPICAL AND ORAL COMPOSITIONS FOR THE TREATMENT OF GUT-SKIN DYSBIOSIS**

(57)    There is disclosed the combined use of an oral probiotic composition with a topical composition for the treatment of gut-skin dysbiosis and related diseases or conditions. The oral probiotic composition comprises different *Lactobacillus* species, preferably the combination of L. plantarum, L. rhamnosus and L. reuteri, optionally further combined with L. acidophilus and the topical composition comprises ectoine. The skin diseases that can be treated with the combined composition include acne, dermatitis, psoriasis, dandruff.

EP 4 151 217 A1

(52) Cooperative Patent Classification (CPC): (Cont.)

C-Sets
A23V 2002/00, A23V 2200/318, A23V 2200/3204,
A23Y 2220/03, A23Y 2220/67, A23Y 2220/71,
A23Y 2220/73

**Description**

The present invention relates to the use of an oral probiotic composition in combination with a topical composition comprising ectoine for the treatment of gut-skin dysbiosis and related diseases or conditions. The invention further relates to a product containing a topical composition comprising ectoine and an oral probiotic composition for combined use in the treatment of gut-skin dysbiosis and related diseases or conditions.

Gut-skin dysbiosis is the unbalanced condition of gut microbiota, which is directly linked to skin microbiota disequilibrium, that can cause different skin diseases like acne, dermatitis, psoriasis, dandruff.

It was surprisingly found that a treatment which combines a probiotic food supplement with a cosmetic cream containing ectoine is suitable for maintaining and protecting skin microbiota homeostasis, and hence to prevent dysbiosis of the skin and the conditions that are associated to such dysbiosis, giving better results than the standard cosmetic treatment alone or in association with the probiotic composition.

BACKGROUND OF THE INVENTION

Skin represents the major organ of the body, with a main role of protection from the external environment. It has been shown that the skin is an active barrier, covered by a number of microorganisms, including bacteria, fungi and viruses (Grice et al., 2011). The skin can be seen as an ecosystem, harbouring microbial communities that live in distinct niches.

Among bacterial phyla, the most presence on the skin are Actinobacteria (51.8%), Firmicutes (24.4%), Proteobacteria (16.5%) and Bacteroidetes (6.3%) (Grice et al., 2009). The main genera of skin microbiota are Corynebacteria, Propionibacteria and Staphylococci (Grice et al., 2009).

The Human Microbiome Project (HMP) of the US National Institutes of Health (NIH) has resulted in microbial identification of communities in 300 healthy individuals across multiple body sites including several skin sites. (Alekseyenko et al., 2013) The project aimed to characterize the human microbiota and its role in health and disease.

Variations in the cutaneous microbiota of healthy individuals are associated with the sebaceous, dry, or moist environments of the skin. In addition, differences in skin microbiota have been associated with factors like age, diet, and gender, climate and geographic location (Perez et al., 2016).

As a complex ecosystem, composed by living and dead microorganisms, the skin can host transient species from the external environment or resident members of skin community. It seems essential that the communities remain in equilibrium for a good status of skin homeostasis. (Grice et al., 2011).

In fact, as skin microbiota activates and educates host immunities, a number of skin inflammatory diseases are associated with shifts in the resident microbiota from a "healthy" to a "disease" state. These diseases can be seen as dysbiotic host-microbial metaorganismal states. (Belkaid et al., 2014).

Differences within skin microbiota can be associated with conditions such as dandruff, acne, psoriasis, atopic dermatitis. (Perez et al., 2016), and also sunburn and vulvovaginitis (Mendling et al., 2016).

For example, it was shown that more than 90% of atopic dermatitis patients are colonised with S. aureus on lesional and non-lesional skin, compared with less than 5% of healthy individuals. Results found suggest that an overabundance of cutaneous S. aureus and, therefore, an associated loss of microbiome diversity, are linked to the pathogenesis of atopic dermatitis (Kobayashi et al., 2015). The effectiveness of antimicrobial agents for the management of skin disorders supports the link between microbes and pathophysiology (Grice et al., 2011). Therapies to maintain healthy skin require the promotion of the growth of symbiotic bacteria more than the inhibition of pathogens growth (Grice et al., 2009).

Other examples of a direct link between skin microbiota and inflammatory diseases are the infections caused by overgrowth of typically commensal bacteria like *Staphylococcus epidermidis*, or *Propionibacterium acnes* (Grice et al., 2011).

Acne is a multifactorial inflammatory disease, interesting the skin where sebaceous glands are more concentrated. Acne can be treated by different therapies, generally by topic or oral treatment, sometimes through antibiotics. These pharmacological treatments can have a number of secondary effects.

It has now been found that the oral administration of probiotics coupled with the topical use of a cream containing ectoine is a safe and efficacious treatment able to restore gut and skin microbiotas balance without side effects.

As the so called gut-skin axis plays a fundamental role as a target point for acne treatment the combined treatment of the invention is particularly useful in the treatment of acne.

Ectoine (CAS number 96702-03-3) is known for its skin osmoprotectant properties (Bownik et al., 2016) and is currently used for the treatment of atopical dermatitis in relation to these properties (Marini et al., 2013). It is also known to be effective in the treatment of inflammatory bowel disease, which is a condition that is also characterised by dysbiosis of the intestine amongst other factors, and this effect appears to be related to the cure of the inflammation within such tissues (Abdel-Aziz et al., 2013).

DETAILED DESCRIPTION OF THE INVENTION

The present invention relates to a probiotic composition containing the following *Lactobacillus* strains:

- *Lactobacillus plantarum*,
- *Lactobacillus rhamnosus*, and
- *Lactobacillus reuteri*

for use in combination with a topical composition comprising ectoine for the prevention or treatment of gut-skin dysbiosis and related skin diseases, wherein the probiotic composition is orally administered to a patient in need thereof and the composition containing ectoine is topically applied onto the skin of the same patient.

The probiotic composition comprises three different probiotic species: *Lactobacillus plantarum* (also known as: *Lactiplantibacillus plantarum*), *Lactobacillus rhamnosus* (also known as: *Lacticaseibacillus rhamnosus*) and *Lactobacillus reuteri* (also known as: *Limosilactobacillus reuteri*), preferably the probiotic composition comprises a probiotic combination comprising *Lactobacillus plantarum* PBS067, *Lactobacillus rhamnosus* LRH020 and *Lactobacillus reuteri* PBS072.

In addition to the three *Lactobacillus* species indicated above, the probiotic composition can optionally contain *Lactobacillus acidophilus* and preferably the *Lactobacillus acidophilus* strain PBS066.

*Lactobacillus plantarum* strain (*L. plantarum* or LP) called "PBS067" was deposited by the applicant at the Leibniz-Institut DSMZ-Deutsche Sammlung von Mikroorganismen und Zellkulturen GmbH, under the Budapest Treaty, on 17/06/2011, under Accession Number "DSM 24937".

*Lactobacillus rhamnosus* strain (*L. rhamnosus* or LRH) called "LRH020" was deposited by the applicant at the Leibniz-Institut DSMZ-Deutsche Sammlung von Mikroorganismen und Zellkulturen GmbH, under the Budapest Treaty, on 17/01/2012, under Accession Number "DSM 25568".

*Lactobacillus reuteri* strain (*L. reuteri* or LR) called "PBS072" was deposited by the applicant at the DSMZ — Deutsche Sammlung von Mikroorganismen und Zellkulturen GmbH under the Budapest Treaty, on 14/092011, under Accession Number "DSM 25175".

*Lactobacillus acidophilus* strain (*L. acidophilus* or *LA*) called "PBS066" was deposited by the applicant at the DSMZ — Deutsche Sammlung von Mikroorganismen und Zellkulturen GmbH under the Budapest Treaty, on 17/06/2011, under Accession Number "DSM 24936".

*Lactobacillus plantarum* (LP), preferably the strain of *Lactobacillus plantarum* PBS067, may be present in the composition in a percentage by weight on the total weight of the probiotic combination from 20% to 50%, preferably from 25% to 45%, even more preferably it is equal to 33%.

*Lactobacillus plantarum* (LP), preferably the strain of *Lactobacillus plantarum* PBS067, may be present in each single unit dose in amounts ranging from 0,5 to 2,5 billion CFU, preferably in amounts ranging from 1 to 2 billion CFU, more preferably it is present in amount of 1 billion CFU.

*Lactobacillus rhamnosus* (LRH), preferably the strain of *Lactobacillus rhamnosus* LRH020, may be present in the composition in a percentage by weight on the total weight of the probiotic combination from 20% to 50%, preferably from 25% to 45%, even more preferably it is equal to 33%.

*Lactobacillus rhamnosus* (LRH), preferably the strain of *Lactobacillus rhamnosus* LRH020 may be present in each single unit dose in amounts ranging from 0,5 a 2,5 billion CFU, preferably in amounts ranging from 1 to 2 billion CFU, more preferably it is present in amount of 1 billion CFU.

*Lactobacillus reuteri* (LR), preferably the strain of *Lactobacillus reuteri* PBS072, may be present in the composition in a percentage by weight on the total weight of the probiotic combination from 20% to 50%, preferably from 25% to 45%, more preferably it is equal to 33%.

*Lactobacillus reuteri* (LR), preferably the strain of *Lactobacillus reuteri* PBS072 may be present in each single unit dose in amounts ranging from 0,5 a 2,5 billion CFU, preferably in amounts ranging from 1 to 2 billion CFU, more preferably it is present in amount of 1 billion CFU.

*Lactobacillus acidophilus* (LA), preferably the strain of *Lactobacillus acidophilus* PBS066, may be present in the composition in a percentage by weight on the total weight of the probiotic combination from 20% to 50%, preferably from 25% to 45%, more preferably it is equal to 33%.

*Lactobacillus acidophilus* (LA), preferably the strain of *Lactobacillus acidophilus* PBS066 may be present in each single unit dose in amounts ranging from 0,5 to 2,5 billion CFU, preferably in amounts ranging from 1 to 2 billion CFU, more preferably it is present in amount of 1 billion CFU.

In one embodiment, the three species of *Lactobacillus plantarum*, *Lactobacillus rhamnosus* and *Lactobacillus reuteri* are present in 1:1:1 ratio or, when *Lactobacillus acidophilus* is added, in a 1:1:1:1 ratio, expressed as CFU.

The probiotic composition comprises *Lactobacillus plantarum* (preferably PBS067), *Lactobacillus rhamnosus* (preferably LRH020), *Lactobacillus reuteri* (preferably PBS072) and optionally *Lactobacillus acidophilus* (PBS066) as active agents, in admixture with at least one physiologically acceptable excipient and/or vehicle.

Preferably the probiotic composition may be administered in the form of tablets or lozenges formulated in a conventional manner. For example, tablets and capsules for oral administration may contain conventional excipients including, but not limited to, binding agents, fillers, lubricants, disintegrants and wetting agents. Tablets may be coated according to methods well known in the art. The compositions of the invention may also be administered as liquid formulations including, but not limited to, aqueous or oily suspensions, solutions, emulsions, syrups, and elixirs. They may also be formulated as a dry product for constitution with water or other suitable vehicle before use. Such liquid preparations may contain additives including, but not limited to, suspending agents, emulsifying agents, nonaqueous vehicles and preservatives.

The topical composition comprising ectoine preferably contains ectoine in a range between 0.1% to 10% by weight.

Preferably ectoine is present in an amount which is below 10%, below 9%, below 8%, below 7%, below 6%, below 5%, below 4%, below 3%, below 2%, below 1% or below 0.5%.

In an embodiment of this invention, the topical composition comprises a weight percentage in ectoine that is selected from the list of 10%, 9%, 8%, 7%, 6%, 5%, 4%, 3.5%, 3%, 2.5%,2.4%, 2.3%, 2.2%, 2.1%, 2%, 1.9%, 1.8%, 1.7%, 1.6%, 1.5%, 1.4%, 1.3%, 1.2%, 1.1%, 1%, 0.9%, 0.8%, 0.7%, 0.6%, 0.5%, 0.4%, 0.3%, 0.2%, 0.1% and any interval of these values, e.g., between 0.5% and 1%, between 0.5% and 2% etc.

In a preferred embodiment, ectoine is produced with a fermentative process from cultures of *Bacillus clausii*, preferably from the *Bacillus clausii* strain ET01 deposited at CGMCC accession No. 16310, as disclosed in WO2020/119828, which is herein incorporated by reference.

The topical composition containing ectoine may be formulated as topical or transdermal formulations comprising aqueous or nonaqueous vehicles including, but not limited to creams, ointments, lotions, pastes, medicated plaster, patch, or membrane.

The probiotic and topical compositions can be administered, independently from one another, one or more times a day, preferably once a day.

In a preferred embodiment, the probiotic composition is administered in a single dose a day, taken in the morning. In another preferred embodiment, the ectoine composition, which is preferably used in the form of a cream, is applied once a day in the morning.

According to a preferred embodiment, skin diseases related to gut-skin dysbiosis are atopic dermatitis, psoriasis, rosacea, dandruff, seborrheic dermatitis, preferably acne.

A further embodiment of the invention relates to the cosmetic use of a probiotic composition containing the following *Lactobacillus* strains:

- *Lactobacillus plantarum*,
- *Lactobacillus rhamnosus*, and
- *Lactobacillus reuteri*

in combination with a topical composition comprising ectoine, for the cosmetic treatment of the skin wherein the probiotic composition is orally administered to a subject in need thereof and the topical composition containing ectoine is topically administered onto the skin of the same subject. In particular, the cosmetic treatment of the skin consists in reducing sebum and/or ameliorating the skin complexion.

A further embodiment of the invention relates to a product or kit containing an oral composition comprising the following probiotic strains:

- *Lactobacillus plantarum*,
- *Lactobacillus rhamnosus*
- *Lactobacillus reuteri*

and, optionally

- *Lactobacillus acidophilus*

and a topical composition comprising ectoine as combined preparation for the prevention or treatment of gut-skin dysbiosis and related skin diseases.

Examples

Example 1. Probiotic composition

A composition in capsules has been prepared, containing:

| Ingredient | mg/cps |
|---|---|
| *Lactobacillus plantarum* | 14 |
| *Lactobacillus rhamnosus* | 24 |
| *Lactobacillus reuteri* | 35 |
| | |
| Maltodextrins | 55 |
| Magnesium stearate | 7 |
| Silica dioxide | 1 |
| TOT | 136 |

MANUFACTURING METHOD:

1. Weight ingredient one by one on a technical weight scale;
2. Mix the ingredient in a pot;
3. Fill the Capsule size 3 DR in a capsule machine. Example 2. Composition containing ectoine in form of cream A cream has been prepared, containing:

| INGREDIENT | Quantity (% by weight) |
|---|---|
| PHASE A | |
| Aqua | 74,30% |
| Disodium EDTA | 0,10% |
| Glycerin | 1,00% |
| PHASE B | |
| Polyglyceryl-3 Methyl Glucose Distearate | 4,00% |
| Glyceryl Stearate | 2,50% |
| Cetearyl Alcohol | 2,50% |
| *Prunus Amygdalus Dulcis* Oil | 8,30% |
| Coco Caprylate/Caprate | 5,00% |
| PHASE C | |
| Ectoin | 1,00% |
| PHASE D | |
| Aqua, Sodium Benzoate, Potassium Sorbate | 1,00% |
| PHASE E | |
| Parfum | 0,30% |
| | 100,00% |

Characteristics:

1 - Appearance: CREAM
2 - Colour: WHITE
3 - Smell: CHARACTERISTIC
4 - pH:5,0-6,0
5 - Viscosity:30000-50000 cP (s=5;5rpm)
6 - Type of emulsion: O/W

MANUFACTURING METHOD:

1. Phase A was prepared by mixing the ingredients under stirring at 70+/-2°C;
2. Phase B was prepared by mixing the ingredients under stirring at 70+/-2°C;
3. Phase B was added into Phase A and mixed for some minutes;
4. The obtained mixture was cooled down at room temperature and Phase C, D and E were added.

Example 3. Clinical trial

The study was aimed to assess the efficacy and the safety of a complete acne treatment (probiotic composition + ectoine composition) in improving skin appearance on adult subjects affected by active acne, in normalizing the presence of sebum reducing inflammation.

A double-blind randomized placebo/reference product-controlled clinical study was carried out on 80 male and female subjects aged between 18 and 50 years old showing acne severity from 1 to 3 according to IGA (Investigator's Global Assessment) severity scale.

According to a predisposed randomization list included subjects were divided in 4 groups:

- G1: 20 subjects received the ectoine composition + the food supplement placebo
- G2: 20 subjects received the complete active treatment (probiotic composition + ectoine composition)
- G3: 20 subjects received the cosmetic reference product (benchmark present on the market) + the food supplement placebo
- G4: 20 subjects received the cosmetic reference product (benchmark present on the market) + the probiotic composition.

Products:

- Topical composition containing ectoine: cream of example 2
- Reference cosmetic product: Sebium Global (Bioderma)
- Oral probiotic composition: Capsule of example 1
- Food supplement placebo

The topical composition and the reference cosmetic product were applied once a day (in the morning) on the face.

The probiotic composition and the food supplement placebo were taken once a day with a glass water (not sparkling water) away from meals.

The study lasted 56 days. Instrumental evaluations of the skin parameters under study were carried out at baseline (T0) and after 28 days (T28) and 56 days (T56) of products use.

The study was moreover integrated with the Dermatologist clinical analysis and the subjects' self-assessment.

1st PARAMETER: Skin sebum

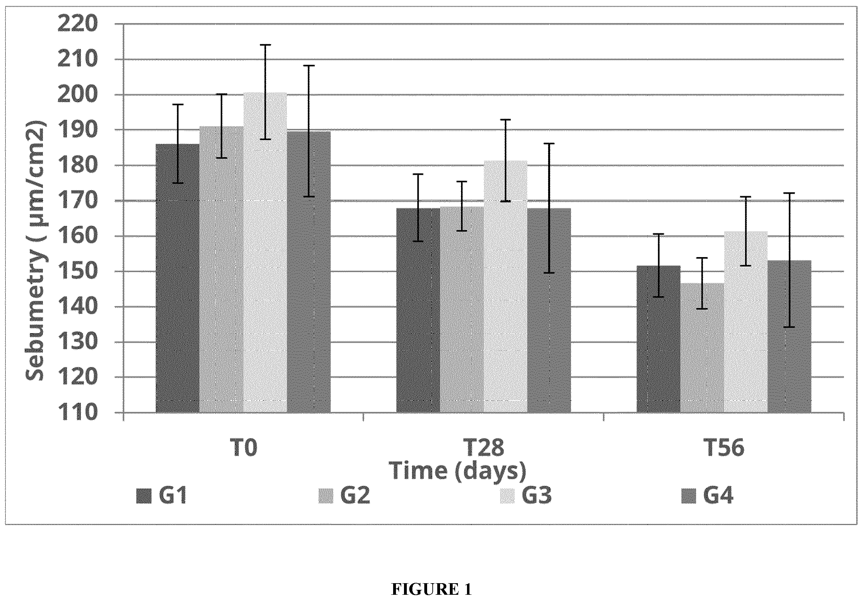

The results of the skin sebum analysis are shown in Figure 1. Based on this data, the following is determined:

$$\Delta(T56\text{-}T0)\ G1 = 35.5$$

$$\Delta(T56\text{-}T0)\ G2 = 44.5$$

$$\Delta(\text{T56-T0})\ \text{G3} = 39.4$$

$$\Delta(\text{T56-T0})\ \text{G4} = 36.5$$

$$\Delta \text{G4} - \Delta \text{G3} = 2.9$$

$$\Delta \text{G2} - \Delta \text{G1} = 10$$

The reductions in the single groups are all statistically significant compared to the placebo.

Comparing the reduction between G2 and G1 and the reduction between G4 and G3, the data show that of the activity of the products targeting skin microbiota (ectoine composition + the food supplement placebo) is a lot higher than the activity of the reference cosmetic products, also when administered in combination with the probiotic composition.

2nd PARAMETER: Skin complexion

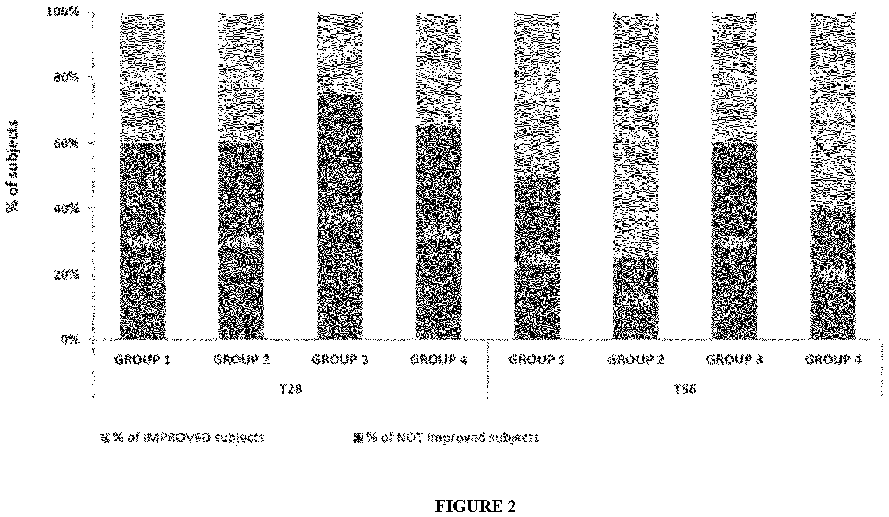

The results are shown in Figure 2.

Skin complexion was evaluated by the dermatologist after 56 days of treatment. As shown in Fig. 2, the improvement of skin complexion in G2 is higher than that in G1 and in G4 is higher than in G3, showing that the use of probiotic composition in combination with a topical product has a better efficacy. Moreover, the improvement in G2 (complete active treatment: probiotic composition + ectoine composition) is the best one, better than the improvement in G3 (cosmetic reference product + the food supplement placebo) and in G4 (cosmetic reference product + the probiotic composition).

3rd PARAMETER: Skin inflammation

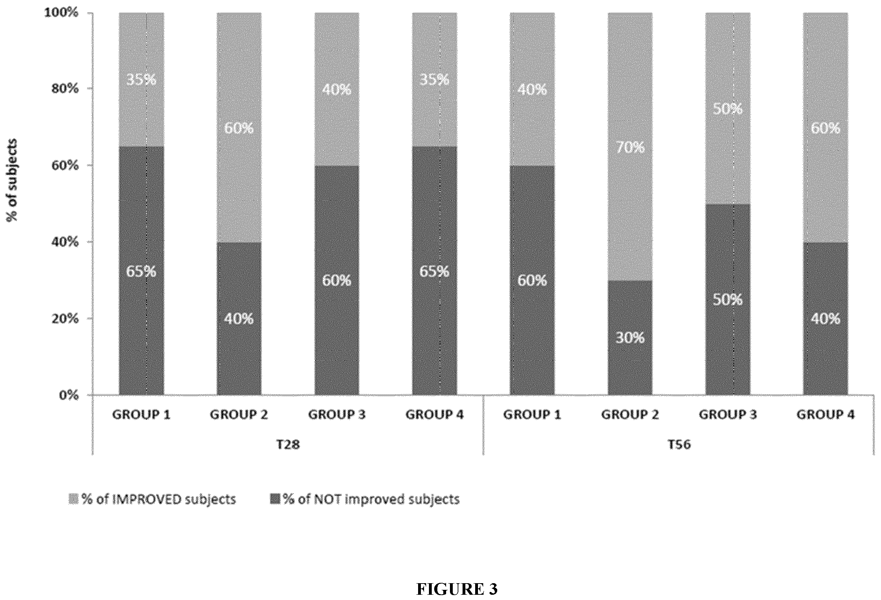

The results are shown in Figure 3.

Skin inflammation was evaluated by the dermatologist after 56 days of treatment.

As shown in Fig. 3, the improvement of skin inflammation in G2 was higher than in the other groups, showing that the use of the probiotic composition in combination with the ectoine topical composition has a better efficacy. In particular, the improvement in G2 (complete active treatment: probiotic composition + ectoine composition) is the best one, better than in G3 (cosmetic reference product + the food supplement placebo) and in G4 (cosmetic reference product + the probiotic composition).

**BIBLIOGRAPHY**

Abdel-Aziz et al., Phytomedicine. 2013 May 15;20(7):585-91. doi: 10.1016/j.phymed.2013.01.009.
Alekseyenko et al., Microbiome. 2013 Dec 23;1(1):31. doi: 10.1186/2049-2618-1-31.
Belkaid et al., Science. 2014 Nov 21;346(6212):954-9. doi: 10.1126/science.
Bownik et al.,Arh Hig Rada Toksikol. 2016 Dec 1;67(4):260-265. doi: 10.1515/aiht-2016-67-2837.
Grice et al., Science. 2009 May 29;324(5931): 1190-2. doi: 10.1126/science.1171700.
Grice et al., Nat Rev Microbiol. 2011 Apr;9(4):244-53. doi: 10.1038/nrmicro2537.
Kobayashi et al.,Immunity. 2015 Apr 21;42(4):756-66. doi: 10.1016/j.immuni.2015.03.014.
Marini et al., Skin Pharmacol Physiol. 2014;27(2):57-65. doi: 10.1159/000351381.
Mendling et al., Adv Exp Med Biol. 2016;902:83-93. doi: 10.1007/978-3-319-31248-4_6.
Perez et al., PLoS One. 2016 Apr 18;11(4):e0151990. doi: 10.1371/journal.pone.0151990.
Schloss et al., PLoS One. 2011;6(12):e27310. doi: 10.1371/journal.pone.0027310.

**Claims**

1.  An oral probiotic composition comprising the following probiotic strains:

- *Lactobacillus plantarum*,
- *Lactobacillus rhamnosus*
- *Lactobacillus reuteri*

and optionally

- *Lactobacillus acidophilus*,

for use in combination with a topical composition comprising ectoine for the prevention or treatment of gut-skin dysbiosis and related skin diseases, wherein the probiotic composition is orally administered to a subject in need thereof and the topical composition comprising ectoine is topically administered onto the skin of the same subject.

2. The oral probiotic composition for use in combination with a topical composition according to claim 1, wherein said probiotic composition comprises *Lactobacillus plantarum* PBS067 deposited at DSMZ Accession No. DSM 24937, *Lactobacillus rhamnosus* LRH020 deposited at DSMZ Accession No. DSM 25568, *Lactobacillus reuteri* PBS072 deposited at DSMZ Accession No. DSM 25175 and optionally *Lactobacillus acidophilus* PBS066 deposited at DSMZ Accession No. DSM 24936.

3. The oral probiotic composition for use in combination with a topical composition according to any one of claims 1-2, wherein said probiotic composition comprises, independently from one another and expressed as a percentage on the total probiotic composition weight:

(i) 20% to 50%, preferably 25% to 45%, more preferably 33% *Lactobacillus plantarum*;
(ii) 20% to 50%, preferably 25% to 45%, more preferably 33% *Lactobacillus rhamnosus*;
(iii) 20% to 50%, preferably 25% to 45%, more preferably 33% *Lactobacillus reuteri*,

and optionally
(iv) 20% to 50%, preferably 25% to 45%, more preferably 33% *Lactobacillus acidophilus.*

4. The oral probiotic composition for use in combination with a topical composition according to any one of claims 1-3, wherein, independently from one another:

(i) the amount of *Lactobacillus plantarum* in a single unit dose ranges from 0,5 to 2,5 billion CFU, preferably from 1 to 2 billion CFU, more preferably it is 1 billion CFU;
(ii) the amount of *Lactobacillus rhamnosus* in a single unit dose ranges from 0,5 to 2,5 billion CFU, preferably from 1 to 2 billion CFU, more preferably it is 1 billion CFU;
(iii) the amount of *Lactobacillus reuteri* in a single unit dose ranges from 0,5 to 2,5 billion CFU, preferably from 1 to 2 billion CFU, more preferably it is 1 billion CFU;

and optionally
(iv) the amount of *Lactobacillus acidophilus* in a single unit dose ranges from 0,5 to 2,5 billion CFU, preferably from 1 to 2 billion CFU, more preferably it is 1 billion CFU.

5. The oral probiotic composition for use in combination with a topical composition according to any one of claims 1-4, wherein *Lactobacillus plantarum*, *Lactobacillus rhamnosus*, *Lactobacillus reuteri* are in a CFU ratio of 1:1:1, and where *Lactobacillus acidophilus* is present, in a 1:1:1:1 ratio.

6. The oral probiotic composition for use in combination with a topical composition according to any one of claims 1-5, wherein said probiotic composition comprises, as active agents, *Lactobacillus plantarum*, preferably the strain PBS067, *Lactobacillus rhamnosus*, preferably the strain LRH020, *Lactobacillus reuteri*, preferably the strain PBS072, and optionally *Lactobacillus acidophilus*, preferably the strain PBS066, in admixture with at least one physiologically acceptable excipient and/or vehicle.

7. The oral probiotic composition for use in combination with a topical composition according to any one of claims 1-6, wherein the topical composition contains ectoine in a range from 0.1% to 10% by weight.

8. The oral probiotic composition for use in combination with a topical composition according to any one of claims 1-7, for the prevention or treatment of a skin disease selected from dermatitis, psoriasis, rosacea, dandruff, seborrheic dermatitis and acne.

9. The use of an oral probiotic composition containing the following *Lactobacillus* strains:

> - *Lactobacillus plantarum*,
> - *Lactobacillus rhamnosus*
> - *Lactobacillus reuteri*

and optionally

> - *Lactobacillus acidophilus*

in combination with a topical composition comprising ectoine for the cosmetic treatment of the skin, wherein the probiotic composition is orally administered to a subject in need thereof and the topical composition comprising ectoine is topically applied onto the skin of the same subject.

10. The use according to claim 9, wherein the cosmetic treatment of the skin consists in reducing sebum and/or improving the skin complexion.

11. A product or kit containing:

> (i) an oral probiotic composition comprising the following *Lactobacillus* strains:

>> - *Lactobacillus plantarum*,
>> - *Lactobacillus rhamnosus*,
>> - *Lactobacillus reuteri*,

> and optionally

>> - *Lactobacillus acidophilus*,

> and
> (ii) a topical composition comprising ectoine,

as a combined preparation for the prevention or treatment of gut-skin dysbiosis and related skin diseases.

FIGURE 1

FIGURE 2

EP 4 151 217 A1

FIGURE 3

EP 4 151 217 A1

## EP 4 151 217 A1

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MICHELOTTI ANGELA ET AL: "Efficacy of a probiotic supplement in patients with atopic dermatitis: a randomized, double-blind, placebo-controlled clinical trial", EUROPEAN JOURNAL OF DERMATOLOGY., vol. 31, no. 2, April 2021 (2021-04), pages 225-232, XP55886899, FR ISSN: 1167-1122, DOI: 10.1684/ejd.2021.4019 | 1,3-11 | INV. A61K31/505 A61K35/741 A61K35/747 A61P17/00 A61P17/04 A61P17/06 A61P17/08 A61P17/10 A61K8/36 A61K8/49 |
| A | * abstract * * page 226, column 1, paragraph 8 * * page 230, column 1, paragraph 3 - page 231, column 1 * | 2 | |
| Y | DE 20 2007 004981 U1 (KLOSTERFRAU MCM VETRIEB GMBH [DE]) 24 April 2008 (2008-04-24) * paragraphs [0002], [0008], [0009], [0021], [0028], [0037]; example 1 * | 1,3-11 | |
| Y | MARINI A ET AL: "Ectoine-Containing Cream in the Treatment of Mild to Moderate Atopic Dermatitis: A Randomised, Comparator-Controlled, Intra-Individual Double-Blind, Multi-Center Trial", SKIN PHARMACOLOGY AND PHYSIOLOGY, S. KARGER AG, BASEL, CH, vol. 27, no. 2, January 2014 (2014-01), pages 57-65, XP009184026, ISSN: 1660-5527, DOI: 10.1159/000351381 * abstract * * page 64 * | 1,3-11 | TECHNICAL FIELDS SEARCHED (IPC) A61K A61P A61Q |
| A | CN 111 528 479 A (SHENZHEN AIMIGENE TECH CO LTD) 14 August 2020 (2020-08-14) * claims 1-5 * | 1,3-11 | |

-/--

The present search report has been drawn up for all claims

| Place of search | Date of completion of the search | Examiner |
|---|---|---|
| Munich | 3 February 2022 | Schlegel, Birgit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 21 19 6947

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | M MILLION ET AL: "Obesity-associated gut microbiota is enriched in Lactobacillus reuteri and depleted in Bifidobacterium animalis and Methanobrevibacter smithii", INTERNATIONAL JOURNAL OF OBESITY, vol. 36, no. 6, June 2012 (2012-06), pages 817-825, XP055622556, London ISSN: 0307-0565, DOI: 10.1038/ijo.2011.153 * table 2 * | | |
| A | ROSENFELDT V ET AL: "EFFECT OF PROBIOTIC LACTOBACILLUS STRAINS IN CHILDREN WITH ATOPIC DERMATITIS", JOURNAL OF ALLERGY AND CLINICAL IMMUNOLOGY, ELSEVIER, AMSTERDAM, NL, vol. 111, no. 2, February 2003 (2003-02), pages 389-395, XP008023585, ISSN: 0091-6749, DOI: 10.1067/MAI.2003.389 * abstract * | 1-11 | |

**TECHNICAL FIELDS SEARCHED (IPC)**

The present search report has been drawn up for all claims

| Place of search | Date of completion of the search | Examiner |
|---|---|---|
| Munich | 3 February 2022 | Schlegel, Birgit |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 2

## ANNEX TO THE EUROPEAN SEARCH REPORT
## ON EUROPEAN PATENT APPLICATION NO.

EP 21 19 6947

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2022

| Patent document cited in search report | Publication date | Patent family member(s) | Publication date |
|---|---|---|---|
| DE 202007004981 U1 | 24-04-2008 | NONE | |
| CN 111528479 A | 14-08-2020 | NONE | |

EPO FORM P0459

For more details about this annex : see Official Journal of the European Patent Office, No. 12/82

## REFERENCES CITED IN THE DESCRIPTION

### Patent documents cited in the description

- WO 2020119828 A

### Non-patent literature cited in the description

- *CHEMICAL ABSTRACTS,* 96702-03-3
- **ABDEL-AZIZ et al.** *Phytomedicine,* 15 May 2013, vol. 20 (7), 585-91
- **ALEKSEYENKO et al.** *Microbiome,* 23 December 2013, vol. 1 (1), 31
- **BELKAID et al.** *Science,* 21 November 2014, vol. 346 (6212), 954-9
- **BOWNIK et al.** *Arh Hig Rada Toksikol,* 01 December 2016, vol. 67 (4), 260-265
- **GRICE et al.** *Science,* 29 May 2009, vol. 324 (5931), 1190-2
- **GRICE et al.** *Nat Rev Microbiol,* April 2011, vol. 9 (4), 244-53
- **KOBAYASHI et al.** *Immunity,* 21 April 2015, vol. 42 (4), 756-66
- **MARINI et al.** *Skin Pharmacol Physiol,* 2014, vol. 27 (2), 57-65
- **MENDLING et al.** *Adv Exp Med Biol,* 2016, vol. 902, 83-93
- **PEREZ et al.** *PLoS One,* 18 April 2016, vol. 11 (4), e0151990
- **SCHLOSS et al.** *PLoS One,* 2011, vol. 6 (12), e27310